(19) Europäisches Patentamt
European Patent Office
Office européen des brevets

(11) **EP 3 960 083 A1**

(12) **EUROPEAN PATENT APPLICATION**

(43) Date of publication:
**02.03.2022 Bulletin 2022/09**

(21) Application number: **21192941.9**

(22) Date of filing: **25.08.2021**

(51) International Patent Classification (IPC):
***A61B 5/367*** (2021.01)    *A61B 5/361* (2021.01)
*A61B 5/363* (2021.01)

(52) Cooperative Patent Classification (CPC):
**A61B 5/367;** A61B 5/361; A61B 5/363;
A61B 5/7267

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **26.08.2020   US 202063070574 P
23.08.2021   US 202117408625**

(71) Applicant: **Biosense Webster (Israel) Ltd
Yokneam, 2066717 (IL)**

(72) Inventors:
• **RAVUNA, Eliyahu**
  **2066717 Yokneam (IL)**
• **YARNITSKY, Jonathan**
  **2066717 Yokneam (IL)**
• **BOTZER, Lior**
  **2066717 Yokneam (IL)**
• **NAKAR, Elad**
  **2066717 Yokneam (IL)**

(74) Representative: **Carpmaels & Ransford LLP
One Southampton Row
London WC1B 5HA (GB)**

(54) **SEPARATING ABNORMAL HEART ACTIVITIES INTO DIFFERENT CLASSES**

(57)    A method that is executed by a determination engine is provided. The method includes receiving one or more pairs of heart beats, generating a model based on the one or more pairs of heart beats, and determining whether two given heart beats are part of a same arrythmia to produce a similarity result for algorithmic input.

EP 3 960 083 A1

**Description**

CROSS-REFERENCE TO RELATED APPLICATIONS

This application claims the benefit of U.S. Provisional Application No. 63/070,574, filed August 26, 2020, the contents of which are incorporated herein by reference.

FIELD OF INVENTION

The present invention is related to an artificial intelligence and machine learning method and system and methods and systems for measuring abnormal heart activities. More particularly, the present invention relates to a machine learning system and method that separates abnormal heart activities into different classes.

BACKGROUND

An electrophysiology (EP) procedure is an assessment of an electrical system or activity of a heart that is used to diagnose an abnormal heartbeat or arrhythmia. EP procedures may be performed utilizing body surface (BS) electrodes and/or inserting one or more catheters through blood vessels into the heart to measure the electrical system or the activity thereof. EP procedures provide heart images (also known as cardiac scans or images), which include images of cardiac tissue, chambers, veins, arteries and/or pathways, based on the measured electrical system or activity.

Some methods in EP procedures (e.g., such as in automated pace mapping, PaSo™ software, intracardiac pattern matching, BS pattern matching, and clustering of the electrocardiogram (ECG) burden) require a conventional correlation algorithm, such as a Pearson product-moment correlation algorithm, to identify whether two heart beats belong to a same arrhythmia or not. Conventional correlation algorithms fail to account for deflection noise, ventricular far field, respiration interference, ringing artifacts of analog or digital filters, and the like, that may imitate a correlation between the two heart beats. Conventional correlation algorithms lack the desired accuracy and consistency. A method and system to reliably measure a strength of an association between two heart beats for any EP procedure would be beneficial.

SUMMARY

A system and method executed by a determination engine includes receiving one or more pairs of heart beats, generating a model based on the one or more pairs of heart beats, and determining whether two given heart beats are part of a same arrythmia to produce a similarity result for algorithmic input. The system and method may be implemented as an apparatus, a system, and/or a computer program product.

BRIEF DESCRIPTION OF THE DRAWINGS

A more detailed understanding may be had from the following description, given by way of example in conjunction with the accompanying drawings, wherein like reference numerals in the figures indicate like elements, and wherein:

FIG. 1 illustrates a diagram of an exemplary system in which one or more features of the disclosed subject matter can be implemented;
FIG. 2 illustrates a block diagram of an example system for remotely monitoring and communicating patient biometrics;
FIG. 3 illustrates a graphical depiction of an artificial intelligence system;
FIG. 4 illustrates a block diagram of a method performed in the artificial intelligence system of FIG. 3;
FIG. 5 illustrates a block diagram of a method according to one or more embodiments;
FIG. 6A illustrates an example of an autoencoder structure for performing a method according to one or more embodiments;
FIG. 6B illustrates a block diagram of a method performed in the autoencoder of FIG; 6A;
FIG. 7 illustrates a screen where the physician selects the cycle length and the ECG Pattern;
FIG. 8 illustrates a block diagram of a method according to one or more embodiments; and
FIG. 9 illustrates the signals obtained during an EP procedure and presentation to select heart beats of the same arrythmia.

DETAILED DESCRIPTION

Disclosed herein is an artificial intelligence and machine learning method and system. More particularly, disclosed is a system and method that measures a strength of an association between two heart beats for any EP procedure. For example, the machine learning algorithm is a processor executable code or software that is necessarily rooted in process operations by, and in processing hardware of, medical device equipment to perform automatic determinations using neural network(s).

According to an embodiment, the machine learning algorithm includes a determination engine that receives EP studies (each of which includes at least two heart beats) and determines for each of the EP studies whether the two heart beats are part of the same arrythmia. The determination engine produces corresponding diagnoses that are further used to generate a model. The model is configured to learn to ignore signal imitations (e.g., deflection noise, ventricular far field, respiration interference, ringing artifacts of analog or digital filters, and the like) when evaluating a pair of heart beats.

According to embodiment, the machine learning algorithm includes a determination engine that receives one or more pairs of heart beats and generates a model based on the one or more pairs of heart beats. The determination engine determines whether two given heart beats are part of a same arrythmia to produce a similarity function for algorithmic input.

The technical effects and benefits of the determination engine include a multi-step manipulation of data respective to the EP studies that produces improved diagnosis information.

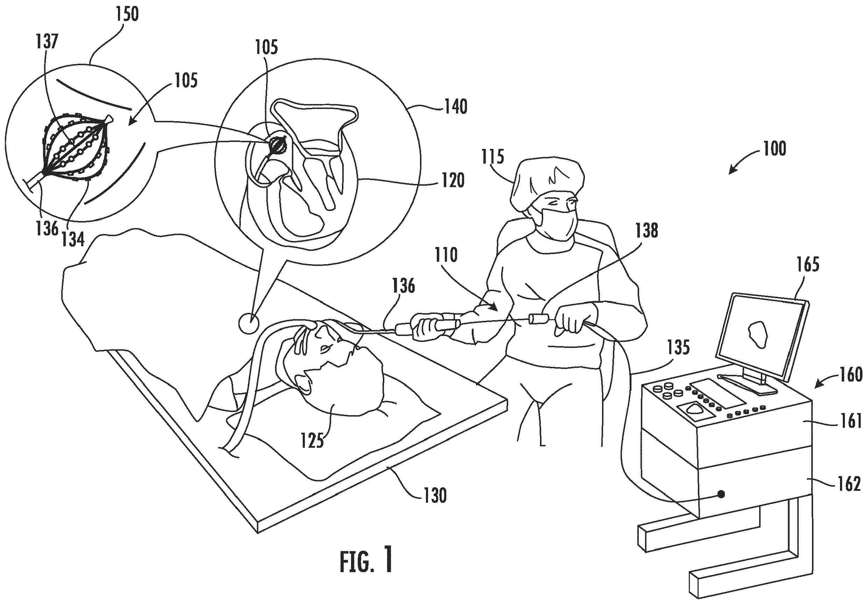

FIG. 1 is a diagram of a system 100 (e.g., medical device equipment) in which one or more features of the subject matter herein can be implemented. All or part of the system 100 may be used to collect information including data of the EP studies, imaging data and/or a training dataset and/or used to implement the machine learning algorithm including the determination engine 101. The system 100, as illustrated, includes a catheter 105 including at least one electrode 106, a probe 110 including a shaft 112, a sheath 113, and a manipulator 114, along with the catheter 105, a physician 115 (or a medical professional), a heart 120, a patient 125, and a bed 130 (or a table). Insets 140 and 150 show the heart 120 and the catheter 105 in greater detail. The system 100 also, as illustrated, includes a console 160 including one or more processors 161 and a memory 162 and a display 165.

Each element and/or item of the system 100 is representative of one or more of that element and/or that item. The example of the system 100 shown in FIG. 1 may be modified to implement the embodiments disclosed herein. The disclosed embodiments may similarly be applied using other system components and settings. Additionally, the system 100 may include additional components, such as elements for sensing electrical activity, wired or wireless connectors, processing and display devices, or the like.

The exemplary system 100 can be utilized to detect, diagnose, and treat cardiac conditions (e.g., using the evaluation engine). Cardiac conditions, such as cardiac arrhythmias (atrial fibrillation in particular), persist as common and dangerous medical ailments, especially in the aging population. In patients (e.g., the patient 125) with normal sinus rhythm, the heart (e.g., the heart 120), which includes atrial, ventricular, and excitatory conduction tissue, is electrically excited to beat in a synchronous, patterned fashion (note that this electrical excitement can be detected as intracardiac signals or the like).

In patients (e.g., the patient 125) with cardiac arrhythmias, abnormal regions of cardiac tissue do not follow the synchronous beating cycle associated with normally conductive tissue as in patients with normal sinus rhythm. Instead, the abnormal regions of cardiac tissue aberrantly conduct to adjacent tissue, thereby disrupting the cardiac cycle into an asynchronous cardiac rhythm (note that this asynchronous cardiac rhythm can also be detected as intracardiac signals). Such abnormal conduction has been previously known to occur at various regions of the heart (e.g., the heart 120), for example, in the region of the sino-atrial (SA) node, along the conduction pathways of the atrioventricular (AV) node, or in the cardiac muscle tissue forming the walls of the ventricular and atrial cardiac chambers.

The catheter 105, which may include the at least one electrode 106 and a catheter needle coupled onto a body thereof, may be configured to obtain biometric data including electrical signals of an intra-body organ, such as the heart 120, and/or to ablate tissue areas of a cardiac chamber of the heart 120. The electrodes 106 are representative of any elements (e.g., such as tracking coils, piezoelectric transducer, electrodes, or combination of elements) configured to ablate the tissue areas or to obtain the biometric data. For example, the catheter 105 may use the electrodes 106 to implement intravascular ultrasound and/or MRI catheterization to image of the heart 120. Inset 150 shows the catheter 105 in an enlarged view, inside a cardiac chamber of the heart 120. Although the catheter 105 is shown to be a point catheter, it will be understood that any shape that includes one or more electrodes 106 may be used to implement the embodiments disclosed herein. Examples of the catheter 106 include, but are not limited to, a linear catheter with multiple electrodes, a balloon catheter including electrodes dispersed on multiple spines that shape the balloon, a lasso or loop catheter with multiple electrodes, or any other applicable shape. Linear catheters may be fully or partially elastic such that it can twist, bend, and or otherwise change its shape based on received signal and/or based on application of an external force (e.g., cardiac tissue) on the linear catheter. The balloon catheter may be designed such that when deployed into a patient's body, its electrodes may be held in intimate contact against an endocardial surface. As an example, a balloon catheter may be inserted into a lumen, such as the pulmonary vein (PV). The balloon catheter may be inserted into the PV in a deflated state, such that the balloon catheter does not occupy its maximum volume while being inserted into the PV. The balloon catheter may expand while inside the PV, such that those electrodes on the balloon catheter are in contact with an entire circular section of the PV. Such contact with an entire circular section of the PV, or any other lumen, may enable efficient imaging and/or ablation.

The probe 110 may be navigated by the physician 115 into the heart 120 of the patient 125 lying on the bed 130 to implement the noted heart imaging. For instance, the physician 115 may insert the shaft 112 through the sheath 113, while manipulating a distal end of the shaft 112 using the manipulator 114 near the proximal end of the catheter 105 and/or deflection from the sheath 113. As shown in an inset 140, the catheter 105 may be fitted at the distal end of the shaft 112. The catheter 105 may be inserted through the sheath 113 in a collapsed state and may be then expanded within the heart 120.

The probe 110, the catheter 105, and other items of the system 100 may be connected to the console 160. The console 160 may include any computing device, which employs the machine learning algorithm represented as the determination engine 101. According to an embodiment, the console 160 includes the one or more processors 161 (a computing hardware) and the memory 162 (a non-transitory tangible media), where the one or more processors 161 execute computer instructions with respect the determination engine 101 and the memory 162 stores these instructions for execution by the one or more processors 161. For instance, the console 160 may be programmed by the determination engine 101 to carry out the functions of receiving one or more pairs of heart beats, generating a model based on the one or more pairs of heart beats, and determining whether two given heart beats are part of a same arrythmia to produce a similarity result for algorithmic input. According to one or more exemplary embodiments, the determination engine 101 may be external to the console 160 and may be located, for example, in the catheter 105, in an external device, in a mobile device, in a cloud-based device, or may be a standalone processor. In this regard, the determination engine 101 may be transferable/downloaded in electronic form, over a network.

The console 160 can be any computing device, as noted herein, including software (e.g., the determination engine 101) and/or hardware, such as a general-purpose computer, with suitable front end and interface circuits for transmitting and receiving signals to and from the catheter 105, as well as for controlling the other components of the system 100. For example, the front end and interface circuits include input/output (I/O) communication interfaces that enables the console 160 to receive signals from and/or transfer signals to the at least one electrode 106. The console 160 may include real-time noise reduction circuitry typically configured as a field programmable gate array (FPGA), followed by an analog-to-digital (A/D) electrocardiograph or electromyogram (EMG) signal conversion integrated circuit. The console 160 may pass the signal from an A/D ECG or EMG circuit to another processor and/or can be programmed to perform one or more functions disclosed herein.

In some embodiments, the console 160 may be further configured to receive and process biometric data and determine if a given tissue area conducts electricity. Biometric data, such as patient biometrics, patient data, or patient biometric data, for example, may include one or more of local time activations (LATs), electrical activity, topology, bipolar mapping, dominant frequency, impedance, or the like. The LAT may be a point in time of a threshold activity corresponding to a local activation, calculated based on a normalized initial starting point (e.g., a reference annotation). Electrical activity may be any applicable electrical signals that may be measured based on one or more thresholds and may be sensed and/or augmented based on signal to noise ratios and/or other filters. A topology may correspond to the physical structure of a body part or a portion of a body part and may correspond to changes in the physical structure relative to different parts of the body part or relative to different body parts. A dominant frequency may be a frequency or a range of frequency that is prevalent at a portion of a body part and may be different in different portions of the same body part. For example, the dominant frequency at an ectopic focus of a heart in an aFib case may be different than the dominant frequency at the healthy tissue of the same heart. Impedance may be the resistance measurement at a given area of a body part.

According to an embodiment, the display 165 is connected to the console 160. During a procedure, the console 160 may facilitate the presentation of a body part rendering to the physician 115 on the display 165, and store data representing the body part rendering in the memory 162. In some embodiments, the physician 115 may be able to manipulate the body part rendering using one or more input devices such as a touch pad, a mouse, a keyboard, a gesture recognition apparatus, or the like. For example, an input device may be used to change a position of the catheter 105, such that rendering is updated. In alternative embodiments, the display 165 may include a touchscreen that can be configured to accept inputs from the medical professional 115 in addition to presenting the body part rendering. The display 165 may be located at a same location or a remote location such as a separate hospital or in separate healthcare provider networks. Additionally, the system 100 may be part of a surgical system that is configured to obtain anatomical and electrical measurements of a patient's organ, such as the heart 120, and performing a cardiac ablation procedure. An example of such a surgical system is the Carto® system sold by Biosense Webster.

According to an embodiment, the console 160 may be connected, by a cable, to body surface electrodes, which may include adhesive skin patches that are affixed to the patient 125. The processor 161, in conjunction with a current tracking module, may determine position coordinates of the catheter 105 inside the body part of the patient 125. The position coordinates may be based on impedances or electromagnetic fields measured between the body surface electrodes and the electrode 106 of the catheter 105 or other electromagnetic components. Additionally, or alternatively, location pads may be located on a surface of the bed 130 and may be separate from the bed 130.

The system 100 may also, and optionally, obtain biometric data such as anatomical measurements of the heart 120 using ultrasound, computed tomography (CT), MRI, or other medical imaging techniques known in the art. The system 100 may obtain ECGs or electrical measurements using catheters or other sensors that measure electrical properties of the heart 120. The biometric data including anatomical and electrical measurements may then be stored in a non-transitory tangible media of the console 160. The biometric data may be transmitted to the computing device 161 from the non-transitory tangible media. Alternatively, or in addition, the biometric data may be transmitted to a server, which may be local or remote, using a network as further described herein.

According to embodiments, the catheter 105 may be configured to ablate tissue areas of a cardiac chamber of the heart 120. Inset 150 shows the catheter 105 in an enlarged view, inside a cardiac chamber of the heart 120. According to embodiments disclosed herein, the ablation electrodes, such as the at least one electrode 106, may be configured to provide energy to tissue areas of an intra-body organ such as heart 120. The energy may be thermal energy and may cause damage to the tissue area starting from the surface of the tissue area and extending into the thickness of the tissue area.

According to one or more embodiments, catheters containing position sensors may be used to determine the trajectory of points on the cardiac surface. These trajectories may be used to infer motion characteristics such as the contractility of the tissue. Maps depicting such motion characteristics may be constructed when the trajectory information is sampled at a sufficient number of points in the heart 120.

Electrical activity at a point in the heart 120 may be typically measured by advancing the catheter 105 containing an electrical sensor at or near its distal tip (e.g., the at least one ablation electrode 134) to that point in the heart 120, contacting the tissue with the sensor and acquiring data at that point. One drawback with mapping a cardiac chamber using the catheter 105 containing only a single, distal tip electrode is the long period of time required to accumulate data on a point-by-point basis over the requisite number of points required for a detailed map of the chamber as a whole. Accordingly, multiple-electrode catheters have been developed to simultaneously measure electrical activity at multiple points in the heart chamber.

According to an example, a multi-electrode catheter may be advanced into a chamber of the heart 120. Anteroposterior (AP) and lateral fluorograms may be obtained to establish the position and orientation of each of the electrodes. EGMs may be recorded from each of the electrodes in contact with a cardiac surface relative to a temporal reference such as the onset of the P-wave in sinus rhythm from a body surface ECG. The system, as further disclosed herein, may differentiate between those electrodes that register electrical activity and those that do not due to absence of close proximity to the endocardial wall. After initial EGMs are recorded, the catheter may be repositioned, and fluorograms and EGMs may be recorded again. An electrical map may then be constructed from iterations of the process above.

electrodes that register electrical activity and those that do not due to absence of close proximity to the endocardial wall. After initial EGMs are recorded, the catheter may be repositioned, and fluorograms and EGMs may be recorded again. An electrical map may then be constructed from iterations of the process above.

According to an example, cardiac mapping may be generated based on detection of intracardiac electrical potential fields. A non-contact technique to simultaneously acquire a large amount of cardiac electrical information may be implemented. For example, a catheter having a distal end portion may be provided with a series of sensor electrodes distributed over its surface and connected to insulated electrical conductors for connection to signal sensing and processing means. The size and shape of the end portion may be such that the electrodes are spaced substantially away from the wall of the cardiac chamber. Intracardiac potential fields may be detected during a single cardiac beat. According to an example, the sensor electrodes may be distributed on a series of circumferences lying in planes spaced from each other. These planes may be perpendicular to the major axis of the end portion of the catheter. At least two additional electrodes may be provided adjacent at the ends of the major axis of the end portion. As a more specific example, the catheter may include four circumferences with eight electrodes spaced equiangularly on each circumference. Accordingly, in this specific implementation, the catheter may include at least 34 electrodes (32 circumferential and 2 end electrodes).

According to another example, an electrophysiological cardiac mapping system and technique based on a non-contact and non-expanded multi-electrode catheter may be implemented. EGMs may be obtained with catheters having multiple electrodes (e.g., between 42 to 122 electrodes). According to this implementation, knowledge of the relative geometry of the probe and the endocardium may be obtained such as by an independent imaging modality such as transesophageal echocardiography. After the independent imaging, non-contact electrodes may be used to measure cardiac surface potentials and construct maps therefrom. This technique may include the following steps (after the independent imaging step): (a) measuring electrical potentials with a plurality of electrodes disposed on a probe positioned in the heart 120; (b) determining the geometric relationship of the probe surface and the endocardial surface; (c) generating a matrix of coefficients representing the geometric relationship of the probe surface and the endocardial surface; and (d) determining endocardial potentials based on the electrode potentials and the matrix of coefficients.

According to another example, a technique and apparatus for mapping the electrical potential distribution of a heart chamber may be implemented. An intra-cardiac multi-electrode mapping catheter assembly may be inserted into a patient's heart 120. The mapping catheter assembly may include a multi-electrode array with an integral reference electrode, or, preferably, a companion reference catheter. The electrodes may be deployed in the form of a substantially spherical array. The electrode array may be spatially referenced to a point on the endocardial surface by the reference electrode or by the reference catheter which is brought into contact with the endocardial surface. The preferred electrode array catheter may carry a number of individual electrode sites (e.g., at least 24). Additionally, this example technique may be implemented with knowledge of the location of each of the electrode sites on the array, as well as knowledge of the cardiac geometry. These locations are preferably determined by a technique of impedance plethysmography.

According to another example, a heart mapping catheter assembly may include an electrode array defining a number of electrode sites. The mapping catheter assembly may also include a lumen to accept a reference catheter having a distal tip electrode assembly which may be used to probe the heart wall. The mapping catheter may include a braid of insulated wires (e.g., having 24 to 64 wires in the braid), and each of the wires may be used to form electrode sites. The catheter may be readily positionable in a heart 120 to be used to acquire electrical activity information from a first set of non-contact electrode sites and/or a second set of in-contact electrode sites.

According to another example, another catheter for mapping electrophysiological activity within the heart may be implemented. The catheter body may include a distal tip which is adapted for delivery of a stimulating pulse for pacing the heart or an ablative electrode for ablating tissue in contact with the tip. The catheter may further include at least one pair of orthogonal electrodes to generate a difference signal indicative of the local cardiac electrical activity adjacent the orthogonal electrodes.

According to another example, a process for measuring electrophysiologic data in a heart chamber may be implemented. The method may include, in part, positioning a set of active and passive electrodes into the heart 120, supplying current to the active electrodes, thereby generating an electric field in the heart chamber, and measuring the electric field at the passive electrode sites. The passive electrodes are contained in an array positioned on an inflatable balloon of a balloon catheter. In embodiments, the array is said to have from 60 to 64 electrodes.

According to another example, cardiac mapping may be implemented using one or more ultrasound transducers. The ultrasound transducers may be inserted into a patient's heart 120 and may collect a plurality of ultrasound slices (e.g., two dimensional or three-dimensional slices) at various locations and orientations within the heart 120. The location and orientation of a given ultrasound transducer may be known and the collected ultrasound slices may be stored such that they can be displayed at a later time. One or more ultrasound slices corresponding to the position of a probe (e.g., a treatment catheter) at the later time may be displayed and the probe may be overlaid onto the one or more ultrasound slices.

According to other examples, body patches and/or body surface electrodes may be positioned on or proximate to a patient's body. A catheter with one or more electrodes may be positioned within the patient's body (e.g., within the patient's heart 120) and the position of the catheter may be determined by a system based on signals transmitted and received between the one or more electrodes of the catheter and the body patches and/or body surface electrodes. Additionally, the catheter electrodes may sense biometric data (e.g., LAT values) from within the body of the patient (e.g., within the heart 120). The biometric data may be associated with the determined position of the catheter such that a rendering of the patient's body part (e.g., heart 120) may be displayed and may show the biometric data overlaid on a shape of the body.

In view of the system 100, it is noted that cardiac arrhythmias, including atrial arrhythmias, may be of a multiwavelet reentrant type, characterized by multiple asynchronous loops of electrical impulses that are scattered about the atrial chamber and are often self-propagating (e.g., another example of intracardiac signals). Alternatively, or in addition to the multiwavelet reentrant type, cardiac arrhythmias may also have a focal origin, such as when an isolated region of tissue in an atrium fires autonomously in a rapid, repetitive fashion (e.g., another example of the intracardiac signals). Ventricular tachycardia (V-tach or VT) is a tachycardia, or fast heart rhythm that originates in one of the ventricles of the heart. This is a potentially life-threatening arrhythmia because it may lead to ventricular fibrillation and sudden death.

For example, aFib occurs when the normal electrical impulses (e.g., another example of the intracardiac signals) generated by the sinoatrial node are overwhelmed by disorganized electrical impulses (e.g., signal interference) that originate in the atrial tissue and/or PVs causing irregular impulses to be conducted to the ventricles. An irregular heartbeat results and may last from minutes to weeks, or even years. aFib is often a chronic condition that leads to a small increase in the risk of death often due to strokes. The first line of treatment for aFib is medication that either slows the heart rate or revert the heart rhythm back to normal. Additionally, persons with aFib are often given anticoagulants to protect them from the risk of stroke. The use of such anticoagulants comes with its own risk of internal bleeding. In some patients, medication is not sufficient and their aFib is deemed to be drug-refractory, i.e., untreatable with standard pharmacological interventions. Synchronized electrical cardioversion may also be used to convert aFib to a normal heart rhythm. Alternatively, aFib patients are treated by catheter ablation.

A catheter ablation-based treatment may include mapping the electrical properties of heart tissue, especially the endocardium and the heart volume, and selectively ablating cardiac tissue by application of energy. Cardiac mapping (which is an example of heart imaging) includes creating a map of electrical potentials (e.g., a voltage map) of the wave propagation along the heart tissue or a map of arrival times (e.g., a LAT map) to various tissue located points. Cardiac mapping (e.g., a cardiac map) may be used for detecting local heart tissue dysfunction. Ablations, such as those based on cardiac mapping, can cease or modify the propagation of unwanted electrical signals from one portion of the heart 120 to another.

The ablation process damages the unwanted electrical pathways by formation of non-conducting lesions. Various energy delivery modalities have been disclosed for forming lesions, and include use of microwave, laser and more commonly, radiofrequency energies to create conduction blocks along the cardiac tissue wall. In a two-step procedure (e.g., mapping followed by ablation) electrical activity at points within the heart 120 is typically sensed and measured by advancing the catheter 105 containing one or more electrical sensors (e.g., electrodes 106) into the heart 120 and obtaining/acquiring data at a multiplicity of points (e.g., ECG data). This ECG data is then utilized to select the endocardial target areas, at which ablation is to be performed.

Cardiac ablation and other cardiac electrophysiological procedures have become increasingly complex as clinicians treat challenging conditions such as atrial fibrillation and ventricular tachycardia. The treatment of complex arrhythmias can now rely on the use of three-dimensional (3D) mapping systems to reconstruct the anatomy of the heart chamber of interest. In this regard, the determination engine 101 employed by the system 100 herein manipulates and evaluates the ECG data to produce improved tissue data that enables more accurate diagnosis, images, scans, and/or maps for treating aFib.

For example, cardiologists rely upon software, such as the Complex Fractionated Atrial Electrograms (CFAE) module of the CARTO® 3 3D mapping system, produced by Biosense Webster, Inc. (Diamond Bar, Calif.), to generate and analyze ECG data. The determination engine 101 of the system 100 enhances this software to generate and analyze the improved tissue data, which further provide multiple pieces of information regarding electrophysiological properties of the heart 120 (including the scar tissue) that represent cardiac substrates (anatomical and functional) of aFib.

Abnormal tissue is generally characterized by low-voltage ECGs. However, initial clinical experience in endo-epicardial mapping indicates that areas of low-voltage are not always present as the sole arrhythmogenic mechanism in such patients. In fact, areas of low or medium voltage may exhibit ECG fragmentation and prolonged activities during sinus rhythm, which corresponds to the critical isthmus identified during sustained and organized ventricular arrhythmias, e.g., applies only to non-tolerated ventricular tachycardias. Moreover, in many cases, ECG fragmentation and prolonged activities are observed in the regions showing a normal or near-normal voltage amplitude (> 1-1.5 mV). Although the latter areas may be evaluated according to the voltage amplitude, they cannot be considered as normal according to the intracardiac signal, thus representing a true arrhythmogenic substrate. The 3D mapping may be able to localize the arrhythmogenic substrate on the endocardial and/or epicardial layer of the right/left ventricle, which may vary in distribution according to the extension of the main disease.

The substrate linked to these cardiac conditions is related to the presence of fragmented and prolonged ECGs in the endocardial and/or epicardial layers of the ventricular chambers (right and left). The 3D mapping system, such as CARTO® 3, is able to localize the potential arrhythmogenic substrate of the cardiomyopathy in terms of abnormal ECG detection.

Electrode catheters (e.g., the catheter 105) are use in medical practice. Electrode catheters are used to stimulate and map electrical activity in the heart and to ablate sites of aberrant electrical activity. In use, the electrode catheter is inserted into a major vein or artery, e.g., femoral artery, and then guided into the chamber of the heart of concern. A typical ablation procedure involves the insertion of a catheter having at least one electrode at its distal end, into a heart chamber. A reference electrode is provided, generally taped to the skin of the patient or by means of a second catheter that is positioned in or near the heart. Radio frequency (RF) current is applied to the tip electrode of the ablating catheter, and current flows through the media that surrounds it, i.e., blood and tissue, toward the reference electrode. The distribution of current depends on the amount of electrode surface in contact with the tissue as compared to blood, which has a higher conductivity than the tissue. Heating of the tissue occurs due to its electrical resistance. The tissue is heated sufficiently to cause cellular destruction in the cardiac tissue resulting in formation of a lesion within the cardiac tissue which is electrically non-conductive. During this process, heating of the electrode also occurs as a result of conduction from the heated tissue to the electrode itself. If the electrode temperature becomes sufficiently high, possibly above 60 degrees Celsius, a thin transparent coating of dehydrated blood protein can form on the surface of the electrode. If the temperature continues to rise, this dehydrated layer can become progressively thicker resulting in blood coagulation on the electrode surface. Because dehydrated biological material has a higher electrical resistance than endocardial tissue, impedance to the flow of electrical energy into the tissue also increases.

Treatments for cardiac conditions such as cardiac arrhythmia often require obtaining a detailed mapping of cardiac tissue, chambers, veins, arteries and/or electrical pathways. For example, a prerequisite for performing a catheter ablation successfully is that the cause of the cardiac arrhythmia is accurately located in the heart chamber. Such locating may be done via an electrophysiological investigation during which electrical potentials are detected spatially resolved with a mapping catheter introduced into the heart chamber. This electrophysiological investigation, the so-called electro-anatomical mapping, thus provides 3D mapping data which can be displayed on a monitor. In many cases, the mapping function and a treatment function (e.g., ablation) are provided by a single catheter or group of catheters such that the mapping catheter also operates as a treatment (e.g., ablation) catheter at the same time. In this case, the determination engine 101 can be directly stored and executed by the catheter 105.

Mapping of cardiac areas such as cardiac regions, tissue, veins, arteries and/or electrical pathways of the heart (e.g., 120) may result in identifying problem areas such as scar tissue, arrhythmia sources (e.g., electric rotors), healthy areas, and the like. Cardiac areas may be mapped such that a visual rendering of the mapped cardiac areas is provided using a display, as further disclosed herein. Additionally, cardiac mapping (which is an example of heart imaging) may include mapping based on one or more modalities such as, but not limited to local activation time (LAT), an electrical activity, a topology, a bipolar voltage mapping, a dominant frequency, or an impedance. Data corresponding to multiple modalities may be captured using a catheter inserted into a patient's body and may be provided for rendering at the same time or at different times based on corresponding settings and/or preferences of a medical professional.

Cardiac mapping may be implemented using one or more techniques. As an example of a first technique, cardiac mapping may be implemented by sensing an electrical property of heart tissue, for example, LAT, as a function of the precise location within the heart. The corresponding data may be acquired with one or more catheters that are advanced into the heart using catheters that have electrical and location sensors in their distal tips. As specific examples, location and electrical activity may be initially measured on hundreds of points on the interior surface of the heart. These data points (e.g., also referred to as electroanatomical points) may be generally sufficient to generate a preliminary reconstruction or map of the cardiac surface to a satisfactory quality. The preliminary map may be combined with data taken at additional points in order to generate a more comprehensive map of the heart's electrical activity. In clinical settings, it is not uncommon to accumulate data a few thousands of points to generate a detailed, comprehensive map of heart chamber electrical activity, by using multi-electrode catheters. The generated detailed map may then serve as the basis for deciding on a therapeutic course of action, for example, tissue ablation, to alter the propagation of the heart's electrical activity and to restore normal heart rhythm.

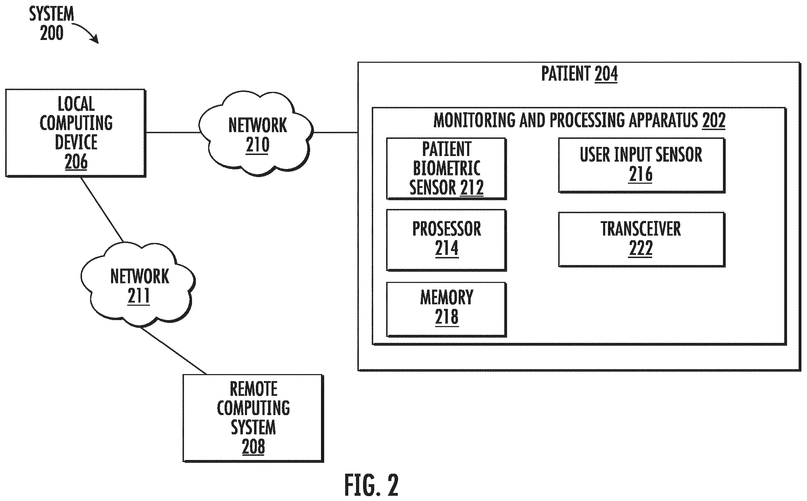

FIG. 2 illustrates a block diagram of an example system 200 for remotely monitoring and communicating biometric data (i.e., patient biometrics, patient data, or patient biometric data) is illustrated. In the example illustrated in FIG. 2, the system 200 includes a monitoring and processing apparatus 202 (i.e., a patient data monitoring and processing apparatus) associated with a patient 204, a local computing device 206, a remote computing system 208, a first network 210, and a second network 211. In accordance with one or more embodiments, the monitoring and processing apparatus 202 can be an example of the catheter 105 of FIG. 1, the patient 204 can be an example of the patient 125 of FIG. 1, and the local computing device 206 can be an example of the console 160 of FIG. 1.

The monitoring and processing apparatus 202 includes a patient biometric sensor 212, a processor 214, a user input (UI) sensor 216, a memory 218, and a transmitter-receiver (i.e., transceiver) 222. In operation, the monitoring and processing apparatus 202 acquires biometric data of the patient 204 (e.g., electrical signals, blood pressure, temperature, blood glucose level or other biometric data) and/or receives at least a portion of the biometric data representing any acquired patient biometrics and additional information associated with any acquired patient biometrics from the one or more other patient biometric monitoring and processing apparatuses. The additional information may be, for example, diagnosis information and/or additional information obtained from an additional device such as a wearable device. The monitoring and processing apparatus 202 may employ the machine learning algorithm and the evaluation engine described herein to process data, including the acquired biometric data as well as any biometric data received from the one or more other patient biometric monitoring and processing apparatuses.

The monitoring and processing apparatus 202 may continually or periodically monitor, store, process, and communicate, via network 210, any number of various patient biometrics (e.g., the acquired biometric data). As described herein, examples of patient biometrics include electrical signals (e.g., ECG signals and brain biometrics), blood pressure data, blood glucose data, and temperature data. The patient biometrics may be monitored and communicated for treatment across any number of various diseases, such as cardiovascular diseases (e.g., arrhythmias, cardiomyopathy, and coronary artery disease) and autoimmune diseases (e.g., type I and type II diabetes).

The patient biometric sensor 212 may include, for example, one or more transducers configured to convert one or more environmental conditions into an electrical signal, such that different types of biometric data are acquired. For example, the patient biometric sensor 212 may include one or more of an electrode configured to acquire electrical signals (e.g., heart signals, brain signals, or other bioelectrical signals), a temperature sensor (e.g., thermocouple), a blood pressure sensor, a blood glucose sensor, a blood oxygen sensor, a pH sensor, an accelerometer, and a microphone.

As described in more detail herein, the monitoring and processing apparatus 202 may implement the machine learning algorithm (e.g., the determination engine 101 of FIG. 1) to receive one or more pairs of heart beats, generate a model based on the one or more pairs of heart beats, and determine whether two given heart beats are part of a same arrythmia to produce a similarity result for algorithmic input. The monitoring and processing apparatus 202 may implement the machine learning algorithm (e.g., the determination engine 101 of FIG. 1) to produce improved tissue data enabling more accurate diagnosis, images, scans, and/or maps for treating aFib.

In another example, the monitoring and processing apparatus 202 may be an ECG monitor for monitoring ECG signals of a heart (e.g., the heart 120 of FIG. 1). In this regard, the patient biometric sensor 212 of the ECG monitor may include one or more electrodes (e.g., electrodes of the catheter 105 of FIG. 1) for acquiring ECG signals. The ECG signals may be used for treatment of various cardiovascular diseases.

The processor 214 may be configured to receive, process, and manage, biometric data acquired by the patient biometric sensor 212, and communicate the biometric data to the memory 218 for storage and/or across the network 210 via the transceiver 222. Data from one or more other monitoring and processing apparatus 202 may also be received by the processor 214 through the transceiver 222, as described in more detail herein. Also, as described in more detail herein, the processor 214 may be configured to respond selectively to different tapping patterns (e.g., a single tap or a double tap) received from the UI sensor 216 (e.g., a capacitive sensor therein), such that different tasks of a patch (e.g., acquisition, storing, or transmission of data) may be activated based on the detected pattern. In some embodiments, the processor 214 can generate audible feedback with respect to detecting a gesture.

The UI sensor 216 includes, for example, a piezoelectric sensor or a capacitive sensor configured to receive a user input, such as a tapping or touching. For example, UI sensor 216 may be controlled to implement a capacitive coupling, in response to tapping or touching a surface of the monitoring and processing apparatus 202 by the patient 204. Gesture recognition may be implemented via any one of various capacitive types, such as resistive capacitive, surface capacitive, projected capacitive, surface acoustic wave, piezoelectric and infrared touching. Capacitive sensors may be disposed at a small area or over a length of the surface, such that the tapping or touching of the surface activates the monitoring device.

The memory 218 is any non-transitory tangible media, such as magnetic, optical, or electronic memory (e.g., any suitable volatile and/or non-volatile memory, such as random-access memory or a hard disk drive).

The transceiver 222 may include a separate transmitter and a separate receiver. Alternatively, the transceiver 222 may include a transmitter and receiver integrated into a single device.

According to an embodiment, the monitoring and processing apparatus 202 may be an apparatus that is internal to a body of the patient 204 (e.g., subcutaneously implantable). The monitoring and processing apparatus 202 may be inserted into the patient 204 via any applicable manner including orally injecting, surgical insertion via a vein or artery, an endoscopic procedure, or a laparoscopic procedure.

According to an embodiment, the monitoring and processing apparatus 202 may be an apparatus that is external to the patient 204. For example, as described in more detail herein, the monitoring and processing apparatus 202 may include an attachable patch (e.g., that attaches to a patient's skin). The monitoring and processing apparatus 202 may also include a catheter with one or more electrodes, a probe, a blood pressure cuff, a weight scale, a bracelet or smart watch biometric tracker, a glucose monitor, a continuous positive airway pressure (CPAP) machine or virtually any device which may provide an input concerning the health or biometrics of the patient.

According to an embodiment, a monitoring and processing apparatus 202 may include both components that are internal to the patient and components that are external to the patient. While a single monitoring and processing apparatus 202 is shown in FIG. 2, example systems may include a plurality of patient biometric monitoring and processing apparatuses. For instance, the monitoring and processing apparatus 202 may be in communication with one or more other patient biometric monitoring and processing apparatuses. Additionally, or alternatively, the one or more other patient biometric monitoring and processing apparatus may be in communication with the network 210 and other components of the system 200.

The local computing device 206 and/or the remote computing system 208, along with the monitoring and processing apparatus 202, can be any combination of software and/or hardware that individually or collectively store, execute, and implement the machine learning algorithm and the evaluation engine and functions thereof. Further, the local computing device 206 and/or the remote computing system 208, along with the monitoring and processing apparatus 202, can be an electronic, computer framework including and/or employing any number and combination of computing device and networks utilizing various communication technologies, as described herein. The local computing device 206 and/or the remote computing system 208, along with the monitoring and processing apparatus 202, can be easily scalable, extensible, and modular, with the ability to change to different services or reconfigure some features independently of others.

According to an embodiment, the local computing device 206 and the remote computing system 208, along with the monitoring and processing apparatus 202, include at least a processor and a memory, where the processor executes computer instructions with respect the machine learning algorithm and the evaluation engine and the memory stores the instructions for execution by the processor.

The local computing device 206 and/or the remote computing system 208, along with the monitoring and processing apparatus 202, can be any combination of software and/or hardware that individually or collectively store, execute, and implement the machine learning algorithm and the evaluation engine and functions thereof. Further, the local computing device 206 and/or the remote computing system 208, along with the monitoring and processing apparatus 202, can be an electronic, computer framework including and/or employing any number and combination of computing device and networks utilizing various communication technologies, as described herein. The local computing device 206 and/or the remote computing system 208, along with the monitoring and processing apparatus 202, can be easily scalable, extensible, and modular, with the ability to change to different services or reconfigure some features independently of others.

According to an embodiment, the local computing device 206 and the remote computing system 208, along with the monitoring and processing apparatus 202, include at least a processor and a memory, where the processor executes computer instructions with respect the machine learning algorithm and the evaluation engine and the memory stores the instructions for execution by the processor.

The local computing device 206 of system 200 is in communication with the monitoring and processing apparatus 202 and may be configured to act as a gateway to the remote computing system 208 through the second network 211. The local computing device 206 may be, for example, a, smart phone, smartwatch, tablet or other portable smart device configured to communicate with other devices via network 211. Alternatively, the local computing device 206 may be a stationary or standalone device, such as a stationary base station including, for example, modem and/or router capability, a desktop or laptop computer using an executable program to communicate information between the processing apparatus 202 and the remote computing system 208 via the PC's radio module, or a USB dongle. Biometric data may be communicated between the local computing device 206 and the monitoring and processing apparatus 202 using a short-range wireless technology standard (e.g., Bluetooth, Wi-Fi, ZigBee, Z-wave and other short-range wireless standards) via the short-range wireless network 210, such as a local area network (LAN) (e.g., a personal area network (PAN)). In some embodiments, the local computing device 206 may also be configured to display the acquired patient electrical signals and information associated with the acquired patient electrical signals, as described in more detail herein.

In some embodiments, the remote computing system 208 may be configured to receive at least one of the monitored patient biometrics and information associated with the monitored patient via network 211, which is a long-range network. For example, if the local computing device 206 is a mobile phone, network 211 may be a wireless cellular network, and information may be communicated between the local computing device 206 and the remote computing system 208 via a wireless technology standard, such as any of the wireless technologies mentioned above. As described in more detail herein, the remote computing system 208 may be configured to provide (e.g., visually display and/or aurally provide) the at least one of the patient biometrics and the associated information to a medical professional, a physician, a healthcare professional, or the like.

In FIG. 2, the network 210 is an example of a short-range network (e.g., local area network (LAN), or personal area network (PAN)). Information may be sent, via short-range network 210, between the monitoring and processing apparatus 202 and the local computing device 206 using any one of various short-range wireless communication protocols, such as Bluetooth, Wi-Fi, Zigbee, Z-Wave, near field communications (NFC), ultraband, Zigbee, or infrared (IR).

The network 211 may be a wired network, a wireless network or include one or more wired and wireless networks, such as an intranet, a local area network (LAN), a wide area network (WAN), a metropolitan area network (MAN), a direct connection or series of connections, a cellular telephone network, or any other network or medium capable of facilitating communication between the local computing device 206 and the remote computing system 208. Information may be sent, via the network 211 using any one of various long-range wireless communication protocols (e.g., TCP/IP, HTTP, 3G, 4G/LTE, or 5G/New Radio). Wired connections may be implemented using Ethernet, Universal Serial Bus (USB), RJ-11 or any other wired connection generally known in the art. Wireless connections may be implemented using Wi-Fi, WiMAX, and Bluetooth, infrared, cellular networks, satellite or any other wireless connection methodology. Additionally, several networks may work alone or in communication with each other to facilitate communication in the network 211. In some instances, the remote computing system 208 may be implemented as a physical server on the network 211. In other instances, the remote computing system 208 may be implemented as a virtual server a public cloud computing provider (e.g., Amazon Web Services (AWS) ®) of the network 211.

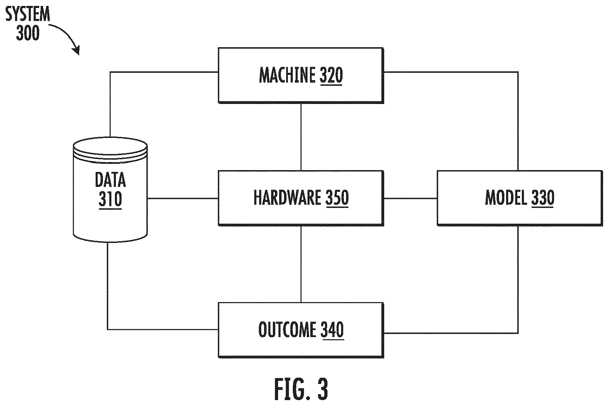

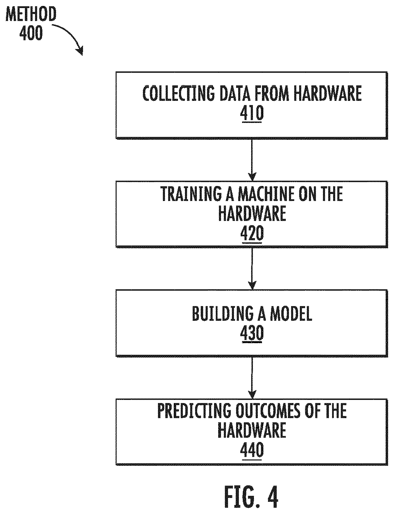

FIG. 3 illustrates a graphical depiction of an artificial intelligence system 300 according to one or more embodiments. The artificial intelligence system 300 includes data 310, a machine 320, a model 330, a plurality of outcomes 340, and underlying hardware 350. FIG. 4 illustrates a block diagram of a method 400 performed in the artificial intelligence system of FIG. 3. The description of FIGS. 3-4 is made with reference to FIG. 2 for ease of understanding.

In general, the artificial intelligence system 300 operates the method 400 by using the data 310 to train the machine 320 (e.g., the local computing device 206 of FIG. 2) while building the model 330 to enable the plurality of outcomes 340 (to be predicted). In such a configuration, the artificial intelligence system 300 may operate with respect to the hardware 350 (e.g., the monitoring and processing apparatus 202 of FIG. 2) to train the machine 320, build the model 330, and predict outcomes using algorithms. These algorithms may be used to solve the trained model 330 and predict outcomes 340 associated with the hardware 350. These algorithms may be divided generally into classification, regression, and clustering algorithms.

At block 410, the method 400 includes collecting the data 310 from the hardware 350. The machine 320 operates as the controller or data collection associated with the hardware 350 and/or is associated therewith. The data 310 may be related to the hardware 350 and can include electrophysiology studies with BS ECG data, IcECG data, location data, and/or position data therein (e.g., biometric data, which may originate with the monitoring and processing apparatus 202 of FIG. 2), which can further include two or more heart beats. For instance, the data 310 may be on-going data, or output data associated with the hardware 350. The data 310 may also include currently collected data, historical data, or other data from the hardware 350. For example, the data 310 may include measurements during a surgical procedure and may be associated with an outcome of the surgical procedure. For example, a temperature of a heart (e.g., of the patient 204 of FIG. 2) may be collected and correlated with an outcome of a heart procedure. Additionally, the data 310 can include signal imitations, such as deflection noise, ventricular far field, respiration interference, ringing artifacts of analog or digital filters, and the like. Additional examples of signal imitations include, but are not limited to, artifacts remaining after a cleaning of a power line hum. These signal imitations will imitate a correlation between the two or more heart beats thereby obscuring whether the two or more heart beats are part of the same arrythmia.

At block 420, the method 400 includes training the machine 320, such as with respect to the hardware 350. The training may include an analysis and correlation of the data 310 collected at block 410. For example, in the case of the two heart beats, the machine 320 may be trained to determine whether the two heart beats are part of a same arrythmia (to produce one or more corresponding diagnoses). That is, the artificial intelligence system 300 can utilize a neural network, as described herein, to determine whether the two heart beats are part of the same arrythmia. According to one or more embodiments, the artificial intelligence system 300 trains the neural network by applying an algorithm to measure a strength of an association between the two heart beats, Thus, unlike the conventional correlation algorithms, the artificial intelligence system 300 can learn other features relevant for the determination of the similarity based on the strength of the association.

At block 430, the method 400 includes building the model 330 on the data 310 associated with the hardware 350 (e.g., generating, by the determination engine 101 of FIG. 1, a model based on the one or more corresponding diagnoses). Building the model 330 may include physical hardware or software modeling, algorithmic modeling, and/or the like. This modeling may seek to represent the data 310 that has been collected and trained. According to an embodiment, the model 330 may be configured to model the operation of hardware 350 and model the data 310 collected from the hardware 350 to predict the outcome achieved by the hardware 350. Further, if the model is trained with high-quality data, the model can learn to ignore signal imitations. The model can be executed by another algorithm, method, device or application, such as an automated pace mapping, the PaSo™ software, a body surface matching, an intracardiac pattern matching, or an arrythmia clustering like the ECG burden charts of the Holter devices, a K-means clustering algorithm. Signal imitations include ringing artifacts of analog and/or digital filters, deflection noise, ventricular far field, respiration interference/artifacts, artifacts remaining after the cleaning of power line hum etc. If the model is used in the context of atrial IcECG, then the ventricular far field can also be considered as a type of signal imitation. The algorithm, method, device, or application that executes the model can include an automated pace mapping, as described in US7907994B2, "*Automated pace-mapping for identification of cardiac arrhythmic conductive pathways and foci*," incorporated herein by reference in its entirety.

At block 440, the method 400 includes predicting the plurality of outcomes 340 of the model 330 associated with the hardware 350 (e.g., a diagnosis). This prediction of the plurality of outcomes 340 may be based on the trained model 330. For example, and to increase understanding of the disclosure, in the case of the heart, if the temperature during the procedure is between 36.5 degrees Celsius and 37.89 degrees Celsius (i.e., 97.7 degrees Fahrenheit and 100.2 degrees Fahrenheit respectively) produces a positive result from the heart procedure, the outcome can be predicted in a given procedure based on the temperature of the heart during the heart procedure. Thus, using the outcome 340 that is predicted, the hardware 350 may be configured to provide a certain desired outcome 340 from the hardware 350.

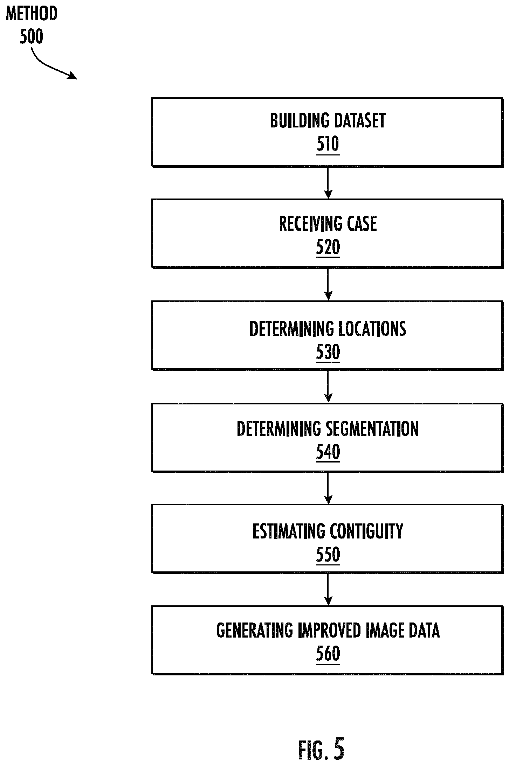

Turning now to FIG. 5, a block diagram of a method 500 is illustrated according to one or more embodiments. In accordance with an embodiment, the method 500 is implemented by the determination engine described herein. Any combination of software and/or hardware (e.g., the console 160 of FIG. 1 and/or the local computing device 206 and the remote computing system 208, along with the monitoring and processing apparatus 202 of FIG. 2) can individually or collectively store, execute, and implement the determination engine and functions thereof.

The method 500 begins at block 510, where the determination engine aggregates/builds a dataset. The dataset includes at least BS ECG and IcECG data of patients. In some cases, the dataset includes validated patient cases where two heart beats correlating to the same arrythmia was verified (e.g., the determination engine verifies the model against a first database after a training to ensure accuracy above a threshold or previous training). For instance, specific EP studies can be manually annotated by a physician to identify whether a case includes the two heart beats that correlate to the same arrythmia.

At block 520, the determination engine trains. More particularly, the determination engine utilizes the dataset of block 510 to train one or more components therein. The training can be performed in real time or retrospectively.

According to one or more exemplary embodiments, the one or more components of the determination engine includes an internal neural network. The inputs to the internal neural network can be a pair of one or more of the BS ECGs, IcECGs, absolute locations, locations relative to a reference catheter, forces exerted by the catheter to the tissue, indications of tissue proximity position inside the respiration cycle, and the like. In this regard, the determination engine utilizes the dataset to train the internal neural network to discover and learn how two heart beats can be considered part of the same arrythmia or not.

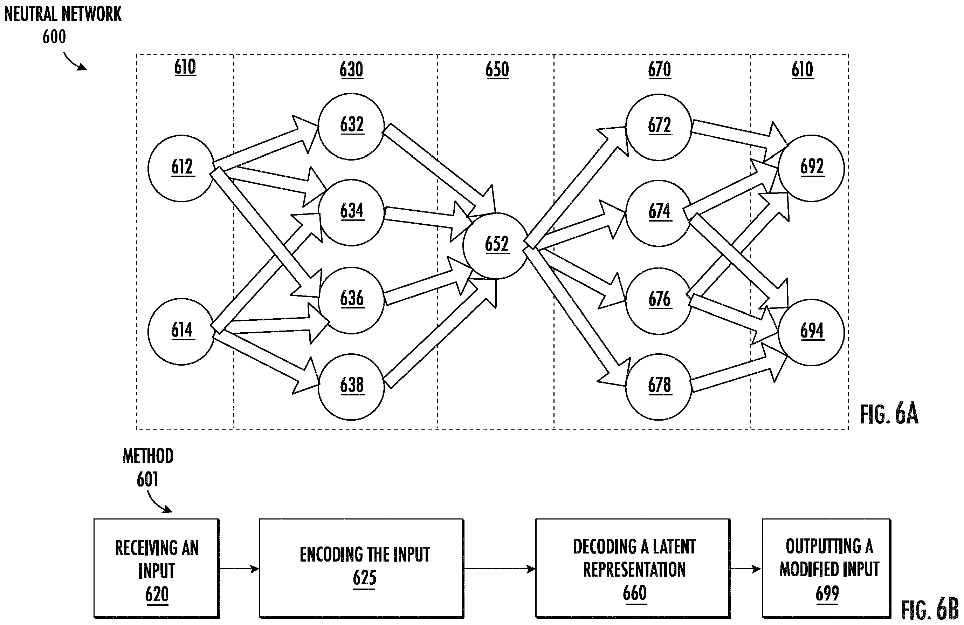

FIG. 6A illustrates an example of an autoencoder architecture 600 and FIG. 6B illustrates a block diagram of a method 601 performed in the autoencoder architecture 600. The autoencoder architecture 600 operates to support implementation of the machine learning algorithm and the evaluation engine described herein. The autoencoder architecture 600 can be implemented in hardware, such as the machine 320 (e.g., the local computing device 206 of FIG. 2) and/or the hardware 350 (e.g., the monitoring and processing apparatus 202 of FIG. 2). Modules 610, 630, 650 of autoencoder 600 collectively operate as a neural network that perform the encoding portion of the autoencoder 600. Modules 850, 670, 610 of autoencoder 600 collectively operate as a neural network that performs the decoding portion of the autoencoder 600. In general, a neural network is a network or circuit of neurons, or in a modern sense, an artificial neural network (ANN), composed of artificial neurons or nodes or cells.

For example, an ANN involves a network of processing elements (artificial neurons) which can exhibit complex global behavior, determined by the connections between the processing elements and element parameters. These connections of the network or circuit of neurons are modeled as weights. A positive weight reflects an excitatory connection, while negative values mean inhibitory connections. Inputs are modified by a weight and summed using a linear combination. An activation function may control the amplitude of the output. For example, an acceptable range of output is usually between 0 and 1, or it could be -1 and 1.

In most cases, the ANN is an adaptive system that changes its structure based on external or internal information that flows through the network. In more practical terms, neural networks are non-linear statistical data modeling or decision-making tools that can be used to model complex relationships between inputs and outputs or to find patterns in data. Thus, ANNs may be used for predictive modeling and adaptive control applications, while being trained via a dataset. Note that self-learning resulting from experience can occur within ANNs, which can derive conclusions from a complex and seemingly unrelated set of information. The utility of artificial neural network models lies in the fact that they can be used to infer a function from observations and also to use it. Unsupervised neural networks can also be used to learn representations of the input that capture the salient characteristics of the input distribution, and more recently, deep learning algorithms, which can implicitly learn the distribution function of the observed data. Learning in neural networks is particularly useful in applications where the complexity of the data or task makes the design of such functions by hand impractical.

Neural networks can be used in different fields. The tasks to which ANNs are applied tend to fall within the following broad categories: function approximation, or regression analysis, including time series prediction and modeling; classification, including pattern and sequence recognition, novelty detection and sequential decision making, data processing, including filtering, clustering, blind signal separation and compression.

Application areas of ANNs include nonlinear system identification and control (vehicle control, process control), game-playing and decision making (backgammon, chess, racing), pattern recognition (radar systems, face identification, object recognition), sequence recognition (gesture, speech, handwritten text recognition), medical diagnosis, financial applications, data mining (or knowledge discovery in databases, "KDD"), visualization and e-mail spam filtering. For example, it is possible to create a semantic profile of user's interests emerging from pictures trained for object recognition.

According to one or more embodiments, the neural network 600 implements a long short-term memory neural network architecture, a CNN architecture, or other the like. The neural network 600 can be configurable with respect to a number of layers, a number of connections (e.g., encoder/decoder connections), a regularization technique (e.g., dropout); and an optimization feature.

The long short-term memory neural network architecture includes feedback connections and can process single data points (e.g., such as images), along with entire sequences of data (e.g., such as speech or video). A unit of the long short-term memory neural network architecture can be composed of a cell, an input gate, an output gate, and a forget gate, where the cell remembers values over arbitrary time intervals and the gates regulate a flow of information into and out of the cell.

The CNN architecture is a shared-weight architecture with translation invariance characteristics where each neuron in one layer is connected to all neurons in the next layer. The regularization technique of the CNN architecture can take advantage of the hierarchical pattern in data and assemble more complex patterns using smaller and simpler patterns. If the neural network 600 implements the CNN architecture, other configurable aspects of the architecture can include a number of filters at each stage, kernel size, a number of kernels per layer.

The neural network 600 may receive as inputs an input pair of beats 610. A form of feature extraction may be performed on the input pairs to identify any number of features in the beat signals before providing to a subsequent module 630 and outputting at module 650.

By way of an alternative example, each beat may be represented by two dimensional CNN including 10 unipolar and 5 bipolar signals with N sample (128 samples may be used). The pairs of similar beats are reconstructed as described herein and fed into the neural network 600. The input may be an image that can be converted into a wavelet or other known transformations as a pre-processing stage to the convolution layer.

Returning to FIG. 5, the process flow 500 continues at block 530, where the determination engine diagnosis a case. The case can be received in real-time, during a heart procedure (e.g., by the console 160 from the catheter 105 of FIG. 1). The case, thus, includes BS ECG and/or IcECG data subsequent to that of the initial BS ECG. That is, the determination engine utilizes the trained neural network to diagnose the case. For instance, the trained neural network of the determination engine is used to automatically determine if two given heart beats of an EP study belong to a same arrhythmia or not. In this regard, the trained neural network ignores signal imitations within the received case. The diagnosis (e.g., a similar/not similar decision) may be used by further algorithms, as described herein. For example, with respect to an ANN, an output can be a binary result, such as similar/not similar, or a similarity percentage. A consumer of the ANN can determine a threshold to consider whether the two beats are similar. The similarity ratio can be calculated by the ANN and displayed on a computer screen as-is, so that a decision is left to a discretion of a physician.

At block 540, the determination engine receives direct feedback. Further, the determination engine can receive feedback identifying whether the diagnosis of block 530 is correct (e.g., a physician is able to correct mistakes of the determination engine, and the determination engine can improve itself according to these corrections.

In some EP systems, such as CARTO® System 3 of Biosense Webster, Inc., the physicians are required to assign beats that belong the same arrhythmia to the same map. If the EP system assigns a beat to a map incorrectly, the physician is required to remove this beat from the map as part of his regular workflow. In an embodiment, this action of the physician, may be used as an input to the machine learning algorithm to train the model. For example, if the beats that the physician removed from the map is the set R = {$R_1$, $R_2$, ..., $R_m$} and the beats remaining in the map is the set M = {$M_1$, $M_2$, ..., $M_n$}, some or all the pairs of the cartesian product of the sets M and R, namely {{$M_1$, $R_1$}, {$M_1$, $R_2$}, ...,{$M_n$, $R_{m-1}$}, {$M_n$, $R_m$}}, can be fed to the machine learning model with an expected similarity of zero, or some other low value. Some or all the pairs of the 2-element combinations of the set M, namely {{$M_1$, $M_2$}, {$M_1$, $M_3$}, ..., {$M_{n-2}$, $M_n$}, {$M_{n-1}$, $M_n$}}, can be fed to the machine learning with an expected similarity of 1, or some other high value. This causes the machine learning model to learn that all remaining beats in the map are similar to each other, and all beats removed from the map are dissimilar to the beats remaining in the map.

The physician may remove some beats not because of the dissimilarity of the arrhythmia, but because of some other reason, such as not enough contact with the tissue, or too much noise, or the like. However, for most practical applications, even if the physician removes the beat for some other reason, a machine learning algorithm that imitates the physician by giving a low similarity score serves treatment well.

In some the EP systems, the physician may create multiple cardiac maps that belong to the same arrhythmia. According to some embodiments, the union of all maps that belong the same arrhythmia are considered as a single map. The fact that multiple maps belong to the same arrhythmia can be automatically determined from the fact that the physician has chosen the same ECG pattern and/or the same cycle length for the two maps.

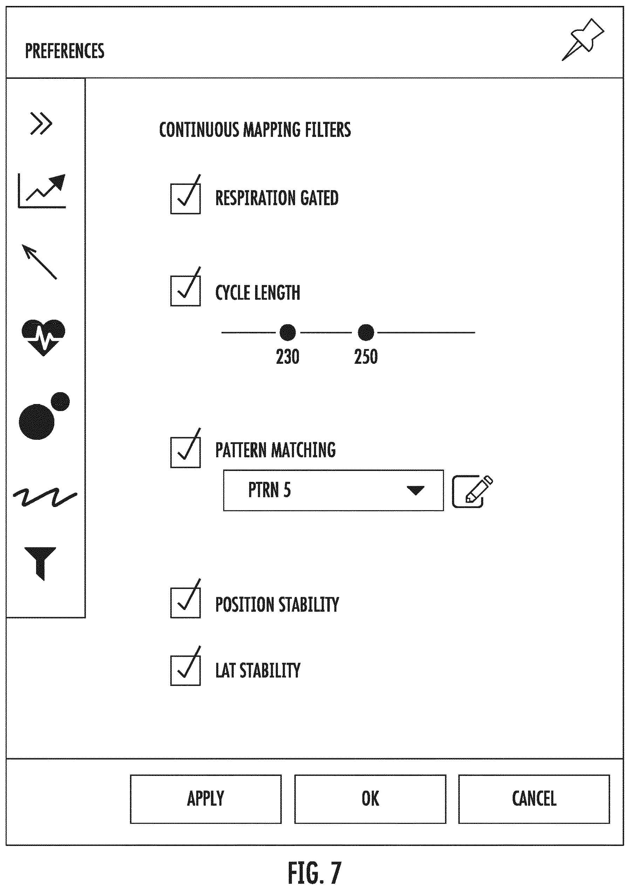

The screen where the physician selects the cycle length and the ECG Pattern, for example in CARTO® SYSTEM 3 of Biosense Webster, Inc. is illustrated in FIG. 7. Maps with the same selected cycle length and the same selected ECG pattern is an indication of the same arrhythmia. FIG. 7 includes a display that illustrates controls related to the filtering of the electro-anatomical points by respiration gating, cycle length, pattern matching, position stability and LAT stability.

At block 550, based on the feedback received at block 540, the determination engine evolves, such that improved heartbeat similarity can be produced on all subsequent EP studies. In turn, the improved heartbeat similarity can be presented to the physician or provided to further algorithms, so that appropriate action can be taken (by the physician) during all subsequent heart procedures.

As shown in FIG. 6B, the method 601 depicts operations of the neural network 600 (e.g., an autoencoder of the determination engine). In the neural network 600, an input layer 610 is represented by a plurality of inputs, such as 612 and 614. With respect to block 620 of the method 601, the input layer 610 receives the plurality of inputs. The plurality of inputs can be BS ECG, along with ultrasound signals, radio signals, audio signal, or a two- or three-dimensional image.

At block 625 of the method 601, the neural network 600 encodes the plurality of inputs utilizing an intracardiac dataset (e.g., the dataset produced by the determination engine in block 510 of FIG. 5) to produce a latent representation. The latent representation includes one or more intermediary images derived from the plurality of inputs. According to one or more embodiments, the latent representation is generated by an element-wise activation function (e.g., a sigmoid function or a rectified linear unit) of the autoencoder of the evaluation engine that applies a weight matrix to the input intracardiac signals and adds a bias vector to the result. The weights and biases of the weight matrix and the bias vector can be initialized randomly, and then updated iteratively during training..

As shown in FIG. 6A, the inputs 612 and 614 are provided to a hidden layer 630 depicted as including nodes 632, 634, 636, and 638. Thus, the transition between layers 610 and 630 can be considered an encoder stage that takes the plurality of inputs 612 and 614 and transfer it to a deep neural network depicted in 630 to learn some smaller representation of the input (e.g., a resulting the latent representation or data coding). The deep neural network could be CNNs, a long short-term memory neural network, a fully connected neural network, or combination thereof. The inputs 612 and 614 can be intracardiac ECG, ECG, or intracardiac ECG and ECG. This encoding provides a dimensionality reduction of the input intracardiac signals. Dimensionality reduction is a process of reducing the number of random variables (of the plurality of inputs) under consideration by obtaining a set of principal variables. For instance, dimensionality reduction can be a feature extraction that transforms data (e.g., the plurality of inputs) from a high-dimensional space (e.g., more than 10 dimensions) to a lower-dimensional space (e.g., 2-3 dimensions). The technical effects and benefits of dimensionality reduction include reducing time and storage space requirements for the data, improving visualization of the data, and improving parameter interpretation for machine learning. This data transformation can be linear or nonlinear. The operations of receiving (block 620) and encoding (block 625) can be considered a data preparation portion of the multi-step data manipulation by the autoencoder of the determination engine.

At block 645 of the method 610, the neural network 600 decodes the latent representation. The decoding stage takes the encoder output (e.g., the resulting the latent representation or data coding) and tries to reconstruct using another deep neural network some form of the inputs 612 and 614. In this regard, the nodes 632, 634, 636, and 638 are combined to produce in the output layer 650 output 652, as shown in block 660 of the method 610. That is, the output layer 690 reconstructs the inputs 612 and 614 on a reduced dimension but without the signal interferences, signal artifacts, and signal noise. Examples of the output 652 include IcECG, clean version of IcECG (denoised version), BS ECG, and denoised ECG.

The neural network 600 performs the processing via the hidden layer 630 of the nodes 632, 634, 636, and 638 to exhibit complex global behavior, determined by the connections between the processing elements and element parameters.

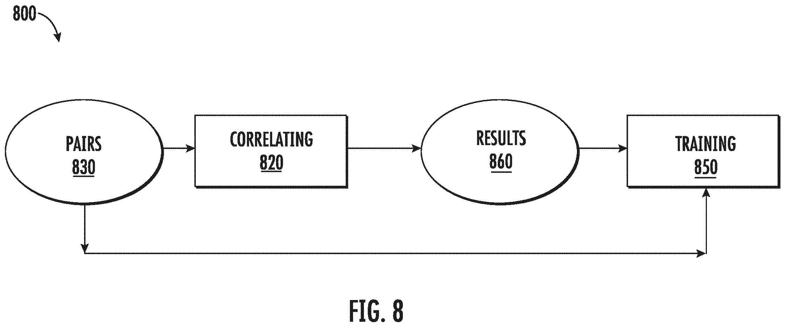

FIG. 8 illustrates a block diagram of a process flow 700 according to one or more embodiments. In accordance with an embodiment, the process flow 700 is implemented by the determination engine described herein. Any combination of software and/or hardware (e.g., the console 160 of FIG. 1 and/or the local computing device 206 and the remote computing system 208, along with the monitoring and processing apparatus 202 of FIG. 2) can individually or collectively store, execute, and implement the determination engine and functions thereof.

At block 820, the determination engine correlates any received pairs of heart beats 830 whose similarities are known. As discussed herein, the model may include an ANN. To train the ANN of the model, the determination engine needs pairs of heart beats whose similarities are known. The known similarities can be either binary, such as 0 for "not similar" and 1 for "similar" or expressed as a percentage, such as real numbers between 0 and 1.

A function f that gives the same value for f(x,y) and f(y,x) is called a commutative function. In an exemplary situation, the neural network takes solely two signals, one signal from a first beat, and a second signal from a second beat. assuming each signal is 1 second sampled in 1K samples per second, the machine learning model should receive 1000 samples from the first beat and 1000 samples form the second beat. If the machine learning model is embodied as an artificial neural network, the neural network should have 2000 scalar inputs. The first inputs may receive the signal of the first beat, and the next 1000 inputs may receive the signal of the second beat. If neural network is naively implemented, the neural network will give a different similarity score for the pair $\{B_1, B_2\}$ and $\{B_2, B_1\}$. To prevent this, and to make the neural network learn to be commutative, a pair is fed to the neural network twice with the same similarity score: Once as $\{B_1, B_2\}$, and once as $\{B_2, B_1\}$. The more the neural network is presented that beats $\{B_1, B_2\}$ and $\{B_2, B_1\}$ are expected to give the same similarity, the better the neural network will learn to act as a commutative function.

Generally, collecting pairs of heart beats with known similarities can require tagging heartbeat pairs manually (e.g., by a physician). According to one or more embodiments, to overcome this problem of requiring a human to tag pairs as similar or not similar, the determination engine can utilize a mathematical algorithm, such as a rule-based mathematical algorithm (e.g., Pearson Product-Moment correlation), to tag the pairs with a similarity percentage. After training (e.g., receiving and correlating) the model with the results of the mathematical algorithm correlation is expected to become approximately as good as the mathematical algorithm correlation.

The inputs to the training, as discussed herein throughout, include two beats ($B_u$ and $B_v$). Further information associated with the two beats may include Pearson Correlation, Temporal alignment of the CS unipolar and bipolar channels, detailed in EP 3 831 304 A1 by YARNITSKY J. at el. Incorporated by reference herein, Position difference of electrodes, Respiration indication, Energy similarity, for example via minimization method defined in the Equation below:

$$\mathrm{ArgMin}\{E = \sum_i \varphi_i E_i\}.$$

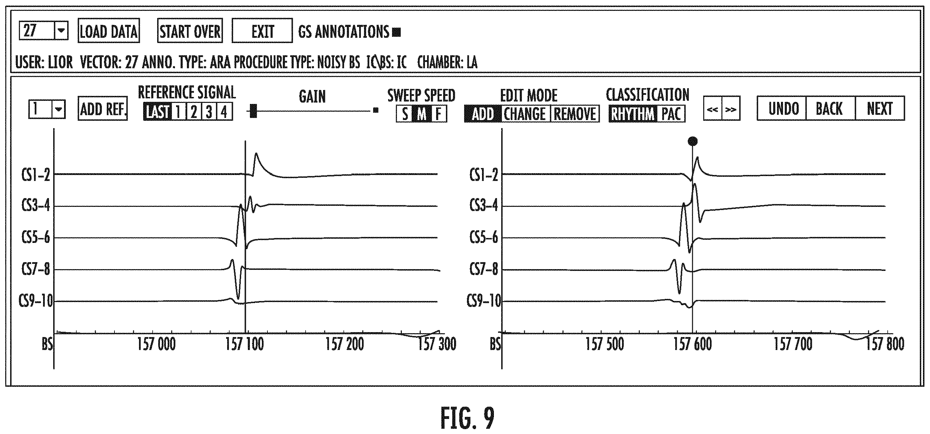

FIG. 9 illustrates an example software application that can be offered to the physicians to enable the physicians to mark which beats belong to the same arrhythmia. An index vertical line may be overlaid over the respective beats on different channels. The database built with such an application may be used to train the machine learning model.

At block 850, the determination engine utilizes the results 860 of the correlation 820 (e.g., an expected output) and the initial pairs 830 as inputs to train the model therein. In this regard, the ANN/model/determination can be deployed to the field to replace conventional algorithms in EP studies (e.g., replace a cross-correlation algorithm in described in US7907994B2, cited herein).

After the initial training of block 850, the ANN/model/determination engine can continuously train to self-improve (e.g., machine learn). In an example, the ANN of the model is deployed to an EP system in a hospital. EP studies are performed by using the ANN/model/determination engine in real-time to calculate pattern matching and the like. Physicians can be given the option to override the decisions of the ANN/model/determination engine. For instance, if the EP system determined that two heart beats are not similar, the physician may be given the option to press on a button to indicate that the two beats are similar, or vice versa. Then, the corrections of the physicians may be uploaded to a central database (e.g., via CD, DVD, memory stick, the Internet, etc.), and the ANN/model/determination engine may be retrained including the corrections of the physicians. As the database includes more and more corrections by the physicians, the ANN/model/determination engine becomes better than the initial training of block 850. The retrained ANN/model/determination engine can be distributed back to the hospitals.

According to one or more exemplary embodiments, to make sure the corrections of the physician make sense, an analyzation can be performed of the corrections visually before adding them to the results 860 and/or the initial pairs 830. This can collection of information can be considered a gold standard database (e.g., a ground truth) that is used after every retraining of the ANN/model/determination engine may be executed against a gold standard database to ensure that an accuracy is above a threshold. Alternatively, it can be required that the accuracy shall be better than the previous accuracy after each retraining.

The technical effects and benefits of the determination engine include a multi-step manipulation of data respective to the EP studies that produces improved diagnosis information.

The flowchart and block diagrams in the figures illustrate the architecture, functionality, and operation of possible implementations of systems, methods, and computer program products according to various embodiments of the present invention. In this regard, each block in the flowchart or block diagrams may represent a module, segment, or portion of instructions, which includes one or more executable instructions for implementing the specified logical function(s). In some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

Although features and elements are described above in particular combinations, one of ordinary skill in the art will appreciate that each feature or element can be used alone or in any combination with the other features and elements. In addition, the methods described herein may be implemented in a computer program, software, or firmware incorporated in a computer-readable medium for execution by a computer or processor. To speed up the training and the inference of the ANN, an AI chip, such as the habana® chips of Intel, may be used. A computer readable medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire

Examples of computer-readable media include electrical signals (transmitted over wired or wireless connections) and computer-readable storage media. Examples of computer-readable storage media include, but are not limited to, a register, cache memory, semiconductor memory devices, magnetic media such as internal hard disks and removable disks, magneto-optical media, optical media such as compact disks (CD) and digital versatile disks (DVDs), a random-access memory (RAM), a read-only memory (ROM), an erasable programmable read-only memory (EPROM or Flash memory), a static random-access memory (SRAM), and a memory stick. A processor in association with software may be used to implement a radio frequency transceiver for use in a WTRU, UE, terminal, base station, RNC, or any host computer.

The terminology used herein is for the purpose of describing particular embodiments only and is not intended to be limiting. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one more other features, integers, steps, operations, element components, and/or groups thereof.

The descriptions of the various embodiments herein have been presented for purposes of illustration, but are not intended to be exhaustive or limited to the embodiments disclosed. Many modifications and variations will be apparent to those of ordinary skill in the art without departing from the scope and spirit of the described embodiments. The terminology used herein was chosen to best explain the principles of the embodiments, the practical application or technical improvement over technologies found in the marketplace, or to enable others of ordinary skill in the art to understand the embodiments disclosed herein.

## Claims

1. A method comprising:

    receiving, by a determination engine executed by one or more processors, one or more pairs of heart beats;
    generating, by the determination engine, a model based on the one or more pairs of heart beats; and
    determining, by the model of the determination engine, whether two given heart beats are part of a same arrythmia to produce a similarity result for algorithmic input.

2. The method of claim 1, wherein the two given heart beats comprise body surface electrocardiogram data, intracardiac electrocardiogram data, absolute location data, location data relative to a reference catheter, force exerted by a catheter to tissue data, indications of tissue proximity position inside respiration cycle.

3. The method of claim 1, wherein the similarity result between the two given heart beats is used by an automated pace mapping, a body surface pattern matching, an intracardiac pattern matching, or an arrythmia clustering retrospectively or in real-time during an electrophysiology study.

4. The method of claim 1, wherein the signal imitations comprise deflection noise, ventricular far field, respiration interference, ringing artifacts of analog or digital filters, or artifacts remaining after a cleaning of a power line hum.

5. The method of claim 1, wherein some or all 2-element permutations of the beats in the map and the beats removed from the map are assumed not to belong to the same arrhythmia for the purpose of the training of the machine learning model.

6. The method of claim 1, wherein the machine learning algorithm is a neural network and every pair is fed to the neural network twice during the training phase. Once as {first beat, second beat}, and once as {second beat, first beat}.

7. A system comprising:

    a memory storing program instruction for a determination engine; and
    one or more processor configured to execute the program instructions to cause the system to:

       receive, by the determination engine, one or more pairs of heart beats;
       generate, by the determination engine, a model based on the one or more pairs of heart beats; and
       determine, by the model of the determination engine, whether two given heart beats are part of a same arrythmia to produce a similarity result for algorithmic input.

8. The method of claim 1 or the system of claim 7, wherein the determination engine utilizes a neural network to determine whether the two given heart beats are part of the same arrythmia.

9. The method or system of claim 8, wherein the determination engine trains the neural network by applying an algorithm to measure a strength of an association between the two given heart beats.

10. The system of claim 9, wherein the training of the neural network by the determination engine occurs in real time or retrospectively.

11. The method of claim 1 or the system of claim 7, wherein the one or more pairs of heart beats comprise known similarities taken from one or more electrophysiology studies.

12. The method of claim 1 or the system of claim 7, wherein the determination engine receives feedback identifying when the determining of whether the two heart beats are part of the same arrythmia is correct.

**13.** The method of claim 1 or the system of claim 7, wherein some or all 2-element combinations of existing EP maps prepared by physicians are assumed to belong to the same arrhythmia for the purpose of the training of the machine learning model.

**14.** The method of claim 1 or the system of claim 7, wherein cardiac EP maps with the same cycle length and/or the same ECG pattern, as selected by the user, are assumed to belong to the same arrhythmia for the purpose of the training of the machine learning model.

FIG. 1

FIG. 2

SYSTEM 200

LOCAL COMPUTING DEVICE 206

NETWORK 210

NETWORK 211

REMOTE COMPUTING SYSTEM 208

PATIENT 204

MONITORING AND PROCESSING APPARATUS 202

PATIENT BIOMETRIC SENSOR 212

USER INPUT SENSOR 216

PROSESSOR 214

TRANSCEIVER 222

MEMORY 218

EP 3 960 083 A1

FIG. 3

METHOD
400

COLLECTING DATA FROM HARDWARE
410

TRAINING A MACHINE ON THE
HARDWARE
420

BUILDING A MODEL
430

PREDICTING OUTCOMES OF THE
HARDWARE
440

FIG. 4

METHOD
500

```
┌─────────────────────────────┐
│      BUILDING DATASET       │
│            510              │
└─────────────────────────────┘
              │
              ▼
┌─────────────────────────────┐
│       RECEIVING CASE        │
│            520              │
└─────────────────────────────┘
              │
              ▼
┌─────────────────────────────┐
│    DETERMINING LOCATIONS    │
│            530              │
└─────────────────────────────┘
              │
              ▼
┌─────────────────────────────┐
│   DETERMINING SEGMENTATION  │
│            540              │
└─────────────────────────────┘
              │
              ▼
┌─────────────────────────────┐
│     ESTIMATING CONTIGUITY   │
│            550              │
└─────────────────────────────┘
              │
              ▼
┌─────────────────────────────┐
│  GENERATING IMPROVED IMAGE DATA │
│            560              │
└─────────────────────────────┘
```

FIG. 5

NEUTRAL NETWORK
600

610        630        650        670        610

612

614

632

634

636

638

652

672

674

676

678

692

694

FIG. 6A

METHOD
601

| RECEIVING AN INPUT 620 | → | ENCODING THE INPUT 625 | → | DECODING A LATENT REPRESENTATION 660 | → | OUTPUTTING A MODIFIED INPUT 699 |

FIG. 6B

EP 3 960 083 A1

PREFERENCES

CONTINUOUS MAPPING FILTERS

☑ RESPIRATION GATED

☑ CYCLE LENGTH

230        250

☑ PATTERN MATCHING

PTRN 5        ▼

☑ POSITION STABILITY

☑ LAT STABILITY

| APPLY | OK | CANCEL |

FIG. 7

800

```
  ┌─────────┐      ┌─────────────┐      ┌─────────┐      ┌─────────────┐
  │  PAIRS  │─────▶│ CORRELATING │─────▶│ RESULTS │─────▶│  TRAINING   │
  │   830   │      │     820     │      │   860   │      │     850     │
  └────┬────┘      └─────────────┘      └─────────┘      └──────▲──────┘
       │                                                        │
       └────────────────────────────────────────────────────────┘
```

FIG. 8

FIG. 9

EP 3 960 083 A1

Europäisches
Patentamt
European
Patent Office
Office européen
des brevets

**EUROPEAN SEARCH REPORT**

Application Number

EP 21 19 2941

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2014/323894 A1 (ZHANG XUSHENG [US] ET AL) 30 October 2014 (2014-10-30) <br> * paragraphs [0029] – [0034] * <br> * paragraphs [0042] – [0046] * <br> * paragraphs [0062] – [0069] * <br> * paragraphs [0078] – [0090] * <br> * figures * <br> ----- | 1-14 | INV. <br> A61B5/367 <br><br> ADD. <br> A61B5/361 <br> A61B5/363 |
| X | US 2018/008203 A1 (IYUN OFRA [IL] ET AL) 11 January 2018 (2018-01-11) <br> * paragraph [0059] * <br> * paragraphs [0071] – [0083] * <br> * figures * <br> ----- | 1-14 | |
| A | US 2009/099468 A1 (THIAGALINGAM ARAVINDA [AU] ET AL) 16 April 2009 (2009-04-16) <br> * paragraphs [0113] – [0114] * <br> * paragraph [0138] * <br> ----- | 8-10 | |
| A | WO 2019/206163 A1 (SHANGHAI MICROPORT EP MEDTECH CO LTD [CN]) 31 October 2019 (2019-10-31) <br> * paragraphs [0069] – [0070] * <br> ----- | 8-10 | TECHNICAL FIELDS SEARCHED (IPC) <br><br> A61B |

The present search report has been drawn up for all claims

| Place of search | Date of completion of the search | Examiner |
|---|---|---|
| The Hague | 12 January 2022 | Bataille, Frédéric |

## ANNEX TO THE EUROPEAN SEARCH REPORT
## ON EUROPEAN PATENT APPLICATION NO.

EP 21 19 2941

This annex lists the patent family members relating to the patent documents cited in the above-mentioned European search report.
The members are as contained in the European Patent Office EDP file on
The European Patent Office is in no way liable for these particulars which are merely given for the purpose of information.

12-01-2022

| Patent document cited in search report | | Publication date | Patent family member(s) | | Publication date |
|---|---|---|---|---|---|
| US 2014323894 | A1 | 30-10-2014 | CN | 105324076 A | 10-02-2016 |
| | | | EP | 2988665 A1 | 02-03-2016 |
| | | | US | 2014323894 A1 | 30-10-2014 |
| | | | WO | 2014176314 A1 | 30-10-2014 |
| US 2018008203 | A1 | 11-01-2018 | AU | 2017203969 A1 | 25-01-2018 |
| | | | CA | 2972476 A1 | 06-01-2018 |
| | | | CN | 107582042 A | 16-01-2018 |
| | | | EP | 3267344 A1 | 10-01-2018 |
| | | | IL | 253136 A | 29-10-2020 |
| | | | JP | 2018000966 A | 11-01-2018 |
| | | | US | 2018008203 A1 | 11-01-2018 |
| US 2009099468 | A1 | 16-04-2009 | EP | 1835852 A1 | 26-09-2007 |
| | | | JP | 2008523929 A | 10-07-2008 |
| | | | US | 2009099468 A1 | 16-04-2009 |
| | | | WO | 2006066324 A1 | 29-06-2006 |
| WO 2019206163 | A1 | 31-10-2019 | AU | 2019258653 A1 | 26-11-2020 |
| | | | CN | 108596132 A | 28-09-2018 |
| | | | EP | 3789911 A1 | 10-03-2021 |
| | | | US | 2021093375 A1 | 01-04-2021 |
| | | | WO | 2019206163 A1 | 31-10-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 63070574
- US 7907994 B2
- EP 3831304 A1, YARNITSKY J.